(19) **Europäisches Patentamt**

**European Patent Office**

**Office européen des brevets**

(11) **EP 1 496 354 A1**

(12) **EUROPEAN PATENT APPLICATION**
published in accordance with Art. 158(3) EPC

(43) Date of publication:
**12.01.2005 Bulletin 2005/02**

(51) Int Cl.7: **G01N 27/327**

(21) Application number: **03743998.1**

(86) International application number:
**PCT/JP2003/002613**

(22) Date of filing: **05.03.2003**

(87) International publication number:
**WO 2003/076919 (18.09.2003 Gazette 2003/38)**

(84) Designated Contracting States:
**AT BE BG CH CY CZ DE DK EE ES FI FR GB GR HU IE IT LI LU MC NL PT RO SE SI SK TR**

(30) Priority: **08.03.2002 JP 2002062963**

(71) Applicant: **MATSUSHITA ELECTRIC INDUSTRIAL CO., LTD.**
**Kadoma-shi, Osaka 571-8501 (JP)**

(72) Inventors:
• **KUWABATA, Susumu**
  **Suita-shi, Osaka 565-0872 (JP)**
• **NAKAMINAMI, Takahiro**
  **Tyonaka-shi, Osaka 560-0033 (JP)**

(74) Representative: **Price, Paul Anthony King et al**
**D Young & Co**
**120 Holborn**
**London EC1N 2DY (GB)**

(54) **SUBSTRATE DETERMINING METHOD**

(57)     There is provided a method for precisely quantitating a substrate by means of a measurement system with a simple structure without occurrence of a measurement error due to an interfering substance. In a method for quantitating a substrate in a sample solution which contains a dissolved interfering substance and the substrate, by the use of an electrode system and a reagent system, (a) a sample solution which contains a dissolved interfering substance and a substrate is supplied to an electrode system comprising a working electrode and a counter electrode under the existence of a reagent system comprising oxidoreductase and an electron mediator; (b) an AC potential is applied to the working electrode, to cause a redox reaction of the electron mediator: (c) an electric signal produced on the basis of the redox reaction is measured by means of the electrode system; and (d) the substrate is quantitated on the basis of the electric signal.

FIG. 1

## Description

Technical Field

The present invention relates to a method for electrochemically quantitating a substrate contained in a sample with the use of oxidoreductase.

Background Art

There have been developed plenty of methods for simple measurement and quantitation of a specific substrate present in a sample. In particular, methods for measurement and quantitation with high selectivity by utilizing a substrate-selective catalytic action of an enzyme has recently been drawing much attention, and some of these methods have been used as methods for quantitating a specific element in body fluid in the field of clinical examinations, and further in the field of self-examinations by ordinary people.

As an example of methods for quantitating and measuring a substrate in a sample, an electrochemical quantitation method of glucose is described. Glucose oxidase (hereinafter abbreviated to GOx) is an enzyme that selectively catalyzes oxidation of glucose. When a certain amount of an oxidized form of electron mediator (compound for transferring electrons, generated due to a reaction, from an enzyme to an electrode) is made present in a reaction solution containing GOx and glucose, glucose oxidation leads to reduction of the oxidized form of electron mediator, to produce a reduced form of electron mediator. The produced reduced form of electron mediator is oxidized by the use of an electrode with a DC potential applied thereto so as to measure a flowing current. Glucose can be quantitated by such a measurement because the flowing current in this case is proportional to an amount of the reduced form of electron mediator produced due to the reaction of GOx with glucose, and the amount of the reduced form of electron mediator is proportional to the glucose content.

Further, a biosensor electrode device can be produced by getting both the enzyme and electron mediator dry on the electrode to be carried. The development of disposable-type glucose sensors based on such technology has recently been attracting a great deal of attention. One representative example is a biosensor described in the specification of Japanese Patent No. 2517153. A disposable-type glucose sensor facilitates measurement of the glucose concentration by simple introduction of a sample solution into a sensor device detachably connected to a measurement device.

While the method for simple measurement and quantitation of a specific substrate present in a sample was described in the above, other than the method for quantitating a substrate, there exists a method as one of general electrochemical measurements, which comprises application of not a DC potential but an AC potential to an electrode, and measurement of an obtained electric signal (hereinafter referred to as an alternating current method). This is a method for primarily obtaining information on a structure of an interface between an electrode and electrolyte, and for example, characteristic evaluations of electrodes carrying active materials in secondary batteries have been conducted using such a measurement method.

Although, as thus described, the use of an enzyme can result in realization of measurement with relatively high selectivity of a substrate, a measurement error has been induced by the influence of other substances than a substrate as a subject to be measured which is contained in a sample in the conventionally-used electrochemical measurement where a DC potential is applied. For example, when an electrochemical measurement is conducted using blood as a sample, easily-oxidizable compounds contained in the blood, such as ascorbic acid (vitamin C), uric acid and acetaminophen, may bring about an error. Such a substance as induces an error in a measurement where the DC potential is applied is called an interfering substance.

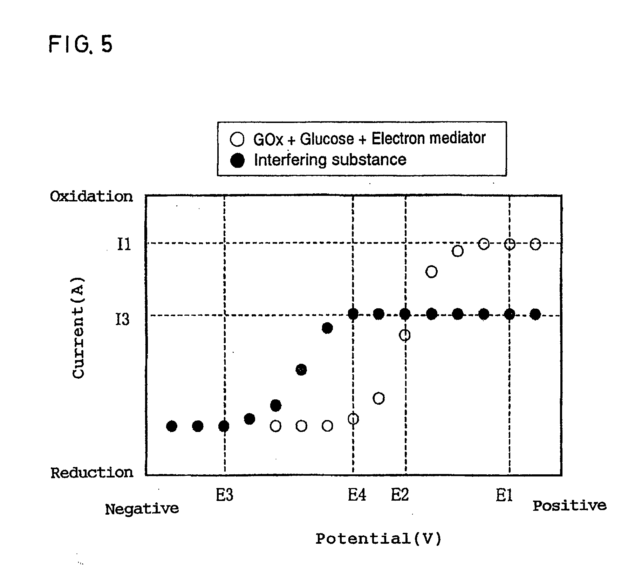

In the following, the reason for occurrence of a current error due to an interfering substance in the conventional method is described, using FIG. 5. FIG. 5 is a graph showing an example of the relationship between the electrode potential and current, obtained in a solution where GOx, an electron mediator and glucose are dissolved, and a solution of an interfering substance. In the glucose quantitation by the conventional method, application of a potential E1, which allows sufficient oxidation of an electron mediator, to an electrode usually generates a current. When E1 is applied to the electrode, electrochemical oxidation of an interfering substance at the electrode proceeds sufficiently, as evident from FIG. 5. Since superimposition of a current (I3) that flows due to the electrochemical oxidation of the interfering substance on a current (I1) due to glucose causes an error in the glucose measurement. Granting that the electron mediator uses a potential to be moderately oxidized, e.g. E2 shown in FIG. 5, a current due to the interfering substance cannot be prevented, and further, a proportion of the current due to the interfering substance in the entire current increases to deteriorate an S/N ratio.

When a compound having redox potential, which is more negative than a potential E3 with which an interfering substance is not oxidized, is used as an electron mediator, there should theoretically be no occurrence of an error. Several attempts have been made to conduct the glucose quantitation using such a compound. In this case, however, a potential difference between GOx and the compound becomes smaller, thereby considerably slowing the transfer rate of electrons therebetween, or preventing the electrons from transferring at all. As a result, problems may arise that a current for the glucose quantitation does not become large enough to be detectable, or it takes extremely long period of time to detect the current, and further the current is not obtained at all.

Moreover, the concentration of an interfering substance in blood differs among individuals, or even in blood of one individual, it differs every day. It is therefore very difficult in the conventional measurement to predict a measurement error that may occur in measuring the interfering substance concentration in blood and then correct it.

A variety of measures have been attempted to correct or remove the influence of an interfering substance. The US Patent No. 6340428 publication for example discloses a method as well as a sensor, in which the influence of an interfering substance is corrected by placement of a third electrode for measurement of an interfering substance in addition to a working electrode and a counter electrode. In advance of progress of an enzyme reaction and a subsequent measurement of a substrate at a working electrode, an interfering substance contained in a sample is measured at a third electrode, whereby a favorable correction has been realized.

As a method for removing the influence of an interfering substance developed has been a method of suppression of a current due to an interfering substance by forming, on an electrode, a film for blocking diffusion of the interfering substance to the electrode. For example, Wang. J et al. has disclosed the use of a poly (o-phenylene diamine) film in "Electroanalysis", August, 1996, Pages 1127-1130.

As thus described, it has been necessary to complexify a structure of a sensor device or an electrode in order to realize correction or removal of the influence of an interfering substance in an electrochemical method using the conventional DC potential, which is used for measurement and quantitation of a specific substrate present in a sample.

In view of the aforesaid drawbacks in the prior art, it is an object of the present invention to provide a method enabling a precise quantitation of a substrate contained in a sample solution by means of a measurement system with a simple structure, without causing a measurement error due to an interfering substance.

Disclosure of Invention

The present invention relates to a method for quantitating a substrate in a sample solution, which contains a dissolved interfering substance and the substrate, by the use of an electrode system and a reagent system, comprising the steps of: (a) supplying a sample solution which contains a dissolved interfering substance and a substrate to an electrode system comprising a working electrode and a counter electrode under the existence of a reagent system comprising oxidoreductase and an electron mediator; (b) applying an AC potential to the working electrode, to cause a redox reaction of the electron mediator; (c) measuring an electric signal produced on the basis of the redox reaction, by means of the electrode system; and (d) quantitating the substrate on the basis of the electric signal.

It is preferable that in the step (a), the working electrode and counter electrode are disposed on the same plane.

It is also preferable that in the step (a), the working electrode and counter electrode are disposed in positions opposed to each other across a space.

It is preferable that the method for quantitating a substrate further comprises a step (e) of applying a DC potential to the working electrode, and a step (f) of measuring an electric signal produced in the step (e).

It is preferable that in the step (b), a central potential of the AC potential is within the range of -0.1 to +0.1 V relative to a redox potential of the electron mediator, and is a potential more positive than a potential that is 0.05 V negative relative to the most negative potential in a potential region where a reaction of the interfering substance at the working electrode is diffusion-controlled.

It is also preferable that the electric signal is impedance.

It is also preferable that the electrode system further comprises a reference electrode.

It is also preferable that the working electrode is a rotating disc electrode or a micro-electrode.

It is preferable that oxidoreductase is glucose oxidase or pyrroloquinoline quinone-dependent glucose dehydrogenase, and the electron mediator is ferrocene carboxylic acid.

It is also preferable that oxidoreductase is pyrroloquinoline quinone-dependent glucose dehydrogenase, and the electron mediator is ruthenium hexacyanate.

Brief Description of Drawings

FIG. 1 is an oblique view of a biosensor used in one example of the present invention, from which a reagent system has been removed.

FIG. 2 is a vertical sectional view (sectional view taken on the line X-X) showing the main part of the biosensor shown in FIG. 1.

FIG. 3 is a graph in which real number elements and imaginary number elements of impedance Z are plotted in an example of the present invention.

FIG. 4 is a diagram showing a construction of one example of measurement devices for use in implementation of a quantitation method of a substrate in accordance with the present invention.

FIG. 5 is a graph showing the relationship between the electrode potential and current, which is obtained with a solution where GOx, an electron mediator and glucose are dissolved and a solution of an interfering substance.

Best Mode for Carrying Out the Invention

A method for quantitating a substrate in accordance with the present invention is characterized by comprising the steps of: (a) supplying a sample solution which contains a dissolved interfering substance and a substrate to an electrode system comprising a working electrode and a counter electrode under the existence of a reagent system comprising oxidoreductase and an electron mediator; (b) applying an AC potential to the working electrode, to cause a redox reaction of the electron mediator; (c) measuring an electric signal produced on the basis of the redox reaction, by means of the electrode system; and (d) quantitating the substrate on the basis of the electric signal.

According to such a method for quantitating a substrate in accordance with the present invention, it is possible to measure a substrate without the influence of an interfering substance. The reason for this is described below.

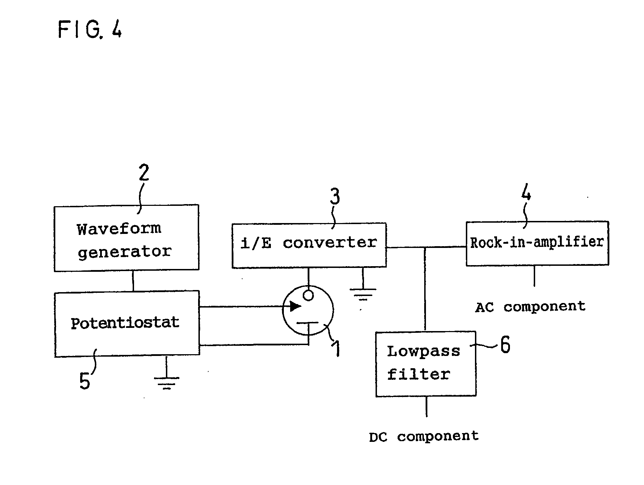

In an alternating current measurement, an AC potential, which uses a certain DC potential as a central potential and contains, by superimposing, an AC potential element with very small amplitude versus the central potential, is applied to a working electrode, and then an obtained electric signal is measured. As a measurement device used for example is the one as shown in FIG. 4.

The measurement device shown in FIG. 4 comprises a waveform generator 2, an i/E converter 3, a rock-in-amplifier 4, a potentiostat 5, and a lowpass filter 6. The waveform generator 2 generates an AC potential and the potentiostat 5 controls a central potential. Subsequently, a current obtained by a reaction at a working electrode in a biosensor 1 is converted into a voltage signal with the i/E converter 3, and an alternate current element and a direct current element are then obtained with the rock-in-amplifier 4 and the lowpass filter 6, respectively. The use of these elements enables quantitation of a substrate, as described later.

The alternating current measurement is described in the following, as comparing with direct current cyclic voltammetry. First considered is direct current cyclic voltammetry in the case of using a sample containing no interfering substance dissolved, and the same electrode system and reagent system as in the method for quantitating a substrate in the above embodiment. In this case, so-called catalytic waves are observed between a current (I) and a potential (E). Further, even in the case of making a potential scanning rate faster, sigmoid waves having no anodic (or cathodic) peak derived from an electron mediator can usually be obtained. When the direct current cyclic voltammetry is performed in such a system that the sigmoid waves can be obtained, by the use of a relatively slow potential scanning rate, almost no hysteresis is observed between the anodic wave and cathodic wave. In this case, therefore, a rate ($dI/dE$) of current variations versus potential variations represents a reciprocal number of a resistance element (R) of the system, substantially in line with the Ohm's law.

Next considered is the case of conducting an alternating current measurement in the same system. As in the aforesaid case of almost no existence of hysteresis in the direct current cyclic voltammetry using a relatively slow potential scanning rate, almost no phase difference occurs between an alternating current ($I_{ac}$) and an AC potential ($E_{ac}$) in an alternating current measurement using a relatively low frequency. Hence impedance (Z) as a resistance element of the system in this case is based on $dI_{ac}/dE_{ac}$. Z is measured, and then the real number elements thereof are plotted as abscissa and the imaginary number elements as ordinate (complex element impedance plotting). There being almost no phase difference between the current and potential at a relatively low frequency, as described above, a plot group exists in the vicinity of the real number axis, having a certain magnitude $|Z_o|$. As the frequency is gradually increased, the magnitude of Z decreases by the influence of the capacity element of the system, and the plot group then forms a curved line that can be approximated by a circular arc with a diameter of $|Z_o|$.

$dI_{ac}/dE_{ac}$ in the alternating current method increases with increasing amount of a substrate contained in the system, as in the case of the direct current cyclic voltammetry. Accordingly, $|Z_o|$ in the complex impedance plot decreases with increasing substrate amount, and as a result, a diameter of an obtained circular arc decreases with increasing substrate amount. It is therefore possible to measure or quantitate an amount of a substrate contained in a sample solution by measurement of an electric signal obtained by the alternating current method. It is preferable here that a central potential of an AC potential to be applied to a working electrode is set in the vicinity of a redox potential of an electron mediator (e.g. in the vicinity of E2 in FIG. 5) because the setting causes increases in $dI_{ac}/dE_{ac}$ and in variation amount of an electric signal accompanied by variations in amount of the substrate.

Meanwhile considered is the case of using an electrolyte singly containing an interfering substance. In this case, in a potential region where the reaction of the interfering substance at an electrode is almost diffusion-controlled (e.g. E4 to E1 in FIG. 5), $dI/dE$ in direct current cyclic voltammetry becomes such a magnitude as is substantially almost ignorable. In the alternating current measurement, therefore, when a central potential of an AC potential to be applied to a working electrode is in the potential range as described above, there is substantially almost no current variation ($dI_{ac}/dE_{ac}$) derived from the interfering substance versus current modulation.

When a plurality of electrochemical reactions are simultaneously proceeding at an electrode, an obtained current is basically the simple sum of currents flowing due to the respective electrochemical reactions. In an electrolyte obtained by combining the two solution compositions in FIG. 5, for example, an obtained current with the potential E1 used is: I = I1 + I3. As thus described, in arguments on the relationship between the current and potential and also R and Z to be obtained from that relationship, even when a sample solution with a substrate and an interfering substance mixed therein is used and the same electrode system and reagent system as in the aforesaid method for quantitating a substrate in the present embodiment are used, both an explanation on the electrolyte containing such a substrate as described above and an explanation on an electrolyte singly containing an interfering substance are established in respect to the substrate and interfering substance, respectively. As a result, for example, $dI_{ac}/dE_{ac}$ in the vicinity of the potential E2 is substantially almost the same as that obtained in an electrolyte containing the aforesaid substrate while not containing an interfering substance, and hence the interfering substance substantially makes almost no contribution to $dI_{ac}/dE_{ac}$.

For the reason described above, according to the method for quantitating a substrate in one embodiment of the present invention, it is possible to quantitate a substrate without the influence of an interfering substance.

Further, in the step (a) of the method for quantitating a substrate in accordance with the present invention, disposition of the working electrode and counter electrode on the same plane is preferred. This makes a simpler substrate quantitation possible.

In this case used may be a biosensor comprising an insulating (first) base plate, an electrode system which comprises a working electrode and a counter electrode that are disposed on the aforesaid (first) base plate, and a reagent system comprising a reagent system which contains oxidoreductase and an electron mediator.

It is also preferable that in the step (a), the working electrode and counter electrode are disposed in positions opposed to each other across a space.

In this case used may be a biosensor comprising an insulating first base plate, an insulating second base plate, an electrode system which comprises a working electrode disposed on the first base plate and a counter electrode disposed on the second base plate, and a reagent system which comprises oxidoreductase and an electron mediator.

It is preferable here that the method for quantitating a substrate in accordance with the present invention further comprises a step (e) of applying a DC potential to the working electrode, and a step (f) of measuring an electric signal produced in the step (e). In such a manner, the electric signal to be measured in the step (f) includes information on the substrate and interfering substance. Accordingly, the combination of this measurement with the alternating current measurement allows quantitation of the interfering substance as well as the substrate, based on the electric signal measured in the step (c) and the electric signal measured in the step (f).

It is also preferable that a central potential of the AC potential is set in the vicinity of a redox potential of the electron mediator. In particular, the central potential is preferably within the range of -0.4 to +0.4 V, and more preferably within the range of -0.1 to +0.1 V, relative to the redox potential of the electron mediator.

It is further preferable that the central potential ($E_{cen}$(V)) of the AC potential is set within, or in the vicinity of, a potential region where the reaction of the interfering substance at the working electrode is diffusion-controlled. It is particularly preferable that the central potential is a potential more positive than a potential that is 0.05 V negative relative to the most negative potential ($E_{min}$(V)) in the potential region where the reaction of the interfering substance at the working electrode is diffusion-controlled. That is, the central potential and the most negative potential preferably satisfy: $E_{cen} > E_{min} - 5$ (V). In this way, the current variation derived from the electron mediator versus modulation of the potential applied to the working electrode can be made greater, while the current variation derived from the interfering substance can be made almost zero. Hence it is possible to completely remove the influence of the interfering substance in the substrate measurement by the alternating current method using the sample solution containing the substrate and interfering substance mixed.

It should be noted that the redox potential of the aforesaid electron mediator and the potential at which the reaction of the interfering substance is diffusion-controlled can be estimated by cyclic voltammetry, wherein a 0.1 M phosphate buffer (pH = 7.0) where the aforesaid substance is dissolved is used as an electrolyte, and a glassy carbon or metal electrode as a working electrode in combination with a suitable reference electrode are used.

Furthermore, the redox potential of the electron mediator carried by the biosensor can be estimated as below. The electron mediator was dissolved and extracted from the biosensor together with a coexisting substance with the use of the aforesaid phosphate buffer to obtain a solution, and the redox potential can then be estimated by cyclic voltammetry, using the obtained solution.

Herein, the AC potential to be applied to the working electrode is a potential (voltage) versus the counter electrode or the reference electrode which is used in combination with the working electrode. Further, an AC potential (voltage) in the present invention may be a potential having a waveform which successively or discretely modulates depending on time "t", and is a concept including an approximate AC potential. More specifically, the AC potential in the present invention may be a potential that satisfies, at least periodically:

$$E(t1) < E(t2) \text{ and } E(t2) > E(t3),$$

or

$$E(t1) > E(t2) \text{ and } E(t2) < E(t3),$$

where $t1 < t2 < t3$. The examples of such waveforms may include sine waves, square waves and step waves.

The electric signal to be used in the method for quantitating a substrate in accordance with the present invention may be an electric signal which varies with the progress of the electrochemical reaction, and may be exemplified by a current, admittance and impedance. In particular, when a potential having sine waves, or step waves which can substantially approximate sine waves, is applied, the electric signal is preferably impedance. While a current and admittance may also be used as the electric signals, it is preferable that these are converted into impedance and then the amount of the substrate is output based on the obtained impedance. In the case of applying a potential with square waves, a current is obtained as the electric signal and, based on dependency (variations) of the electric signal on (with) time and the AC potential, information regarding the substrate amount can be obtained.

It is also preferable that the electrode system further comprises a reference electrode. This arrangement stabilizes a potential to be applied to the working electrode, thereby allowing a more stable substrate measurement. While the reference electrode to be used can be an Ag/AgCl electrode, a saturated calomel electrode (SCE) or the like, it is not limited to these and any electrode with a stable potential may be used.

As for the working electrode to be used in the method for quantitating a substrate in accordance with the present invention, a conventionally-known ones can be used without a specific limitation. It is particularly preferable that the working electrode is a rotating disc electrode (hereinafter abbreviated to RDE) or a micro-electrode. The use of RDE which rotates at a fixed rate, or the use of a micro-electrode which has an electrode area of such a small size that a lateral diffusion of the electron mediator toward the electrode surface contributes to the electrochemical reaction, allows larger current variation derived from the electron mediator versus modulation of the potential applied to the working electrode, compared with the case of using a static bulk electrode. This can thus enhance the sensitivity toward the substrate. The radius of the micro-electrode is preferably from not longer than 50 μm (in the case of a circle electrode), and more preferably from not longer than 50 μm and not shorter than 20 μm. The substrate measurement by means of the alternating current method according to the present invention can be stably conducted by the use of higher frequency, as suggested, in direct current voltammogram using these electrodes, by the fact that hysteresis between the anodic wave and cathodic wave is very small even when no catalyst reaction occurs and when a potential scanning rate is made relatively faster.

As for the sample solution, where an interfering substance is dissolved, to be used in the method for quantitating a substrate in accordance with the present invention, a solution where a substrate and an interfering substance are dissolved, and further a living organism solution where a substrate and an interfering substance are dissolved, e.g. blood, plasma, serum, urine and interstitial fluid, can be used. As for examples of the interfering substance cited can be ascorbic acid (vitamin C), uric acid and acetaminophen.

As for oxidoreductase to be used in the method for quantitating a substrate in accordance with the present invention, a suitable one can be selected according to the type of substrate to dissolve in a sample solution as an object to be measured. In a case where the substrate as an object to be measured is glucose, for example, oxidoreductase may be exemplified by glucose oxidase, pyrroloquinoline quinone-dependent glucose dehydrogenase, nicotinamide-adenine dinucleotide-dependent glucose dehydrogenase, and nicotinamide adenine dinucleotide phosphate-dependent glucose dehydrogenase; in a case where the substrate is cholesterol, oxidoreductase may be exemplified by cholesterol oxidase, nicotinamide-adenine dinucleotide-dependent cholesterol dehydrogenase, and nicotinamide adenine dinucleotide phosphate-dependent cholesterol dehydrogenase. Other than the aforesaid oxidoreductases, for example, alcohol dehydrogenase, lactate oxidase, xanthine oxidase, amino acid oxidase, ascorbic acid oxidase, acyl-CoA oxidase, uricase, glutamate dehydrogenase, fructose dehydrogenase, and the like, can be used according to the type of substrate as an object to be measured.

As examples of the electron mediator to be used in the method for quantitating a substrate in accordance with the present invention, metal complexes such as a ferrocene derivative, ferri/ferrocyanide ions, ruthenium hexacyanate, osmium-tris(bipyridynium) and osmium-di(bipyridynium)imidazolium, a quinone derivative such as p-benzoquinone, a phenazinium derivative such as phenazine methosulfate, a phenothiazinium derivative such as methylene blue, nicotinamide adenine dinucleotide, and nicotinamide adenine dinucleotide phosphate may be cited.

An electron mediator having a high electron transfer rate against an enzyme to be used can be used favorably in combination with the enzyme. Among such electron mediators preferred are a ferrocene derivative, ruthenium hexacyanate, osmium-tris(bipyridynium) and osmium-di(bipyridynium)imidazolium, which are highly stable electron mediators.

Among them, electron mediators having a relatively high redox potential are particularly preferred in implementation of the present invention in that electric signals based respectively on a substrate and an interfering substance, which are obtained in application of an AC potential, can be obtained in a favorably separated manner.

These electron mediators may be in a linked form with a polymer backbone, or in such a form that part or the whole thereof forms polymer chains. Further, oxygen can be used as the electron mediator. One type or two of the above examples are used as the electron mediator.

In the following, the present invention is more specifically described using examples; however the present invention is not limited thereto.

Example 1

10 mL of a phosphate buffer with pH 7, where 0.2 mM of ferrocene carboxylic acid and 4 μM of glucose oxidase were dissolved as a reagent system, was obtained. This 10 mL phosphate buffer was put into a container made of Pyrex Glass to be used as an electrolyte, and a circular platinum disc with a diameter of 3 mm was used as a working electrode while a 2 cm-square platinum plate was used as a counter electrode, to construct an electrochemical cell. A glucose aqueous solution as a sample solution where ascorbic acid was dissolved was added such that the concentrations of ascorbic acid and glucose were 0.5 mM and 20 mM (about 400 mg/ dL), respectively.

Then, after the elapse of a certain period of time, an AC potential, having a central potential of +0.1 V relative to the counter electrode and the amplitude of 0.01 V, was applied to the working electrode. A central potential at this working electrode was within the range of -0.1 V to +0.1 V relative to a redox potential of ferrocene carboxylic acid. And the central potential was a potential more positive than a potential that was 0.05 V negative (0.13 V (pH 7) vs Ag/AgCl) relative to the most negative potential (0.18 V (pH 7) vs Ag/AgCl) in a potential region where the reaction of ascorbic acid at the working electrode is diffusion-controlled. The frequency of the AC potential was successively varied from 16 mHz to 10 kHz, to be more precise, the value of $(1.6, 2.5, 4.0, 6.3 \text{ or } 10) \times (10^{-2}, 10^{-1}, 1, 10, 10^2 \text{ or } 10^3)$ Hz was used.

A certain period of time after the application of the AC potential, impedance Z was measured to plot complex impedance. As thus described, since there was almost no phase difference between the current and potential at a relatively low frequency, the plot appeared in the vicinity of the real number axis; as the frequency was gradually increased, the magnitude of Z became smaller and the plot group formed an almost circular arc.

Next, series of the same electrolytes as thus described were prepared by varying the glucose concentration. The ascorbic acid concentration was fixed to be 0.5 mM and the respective glucose concentrations were 2, 3, 4, 5 and 10 mM. Using these electrolytes, the measurement and plotting were conducted in the same manner as above. As the electrolytes with the lower glucose concentrations were used, the diameter of the obtained circular arc gradually became longer, while having a certain correlation with the glucose concentration. Such a behavior was also observed in a case where a glucose aqueous solution containing no ascorbic acid was used as a sample solution.

It was therefore possible, by previously preparing an analytical calibration curve relating a diameter of a circle arc in an obtained complex impedance plot with a glucose concentration, to determine the glucose aqueous solution concentration, without the influence of ascorbic acid, from a diameter of a circular arc of complex impedance plot having been obtained in terms of a glucose aqueous solution with an unknown concentration. In this wise, the use of the method for quantitating a substrate in accordance with the present invention allowed precise quantitation of a substrate contained in a sample solution in a simply-structured measurement system without occurrence of a measurement error due to an interfering substance.

Example 2

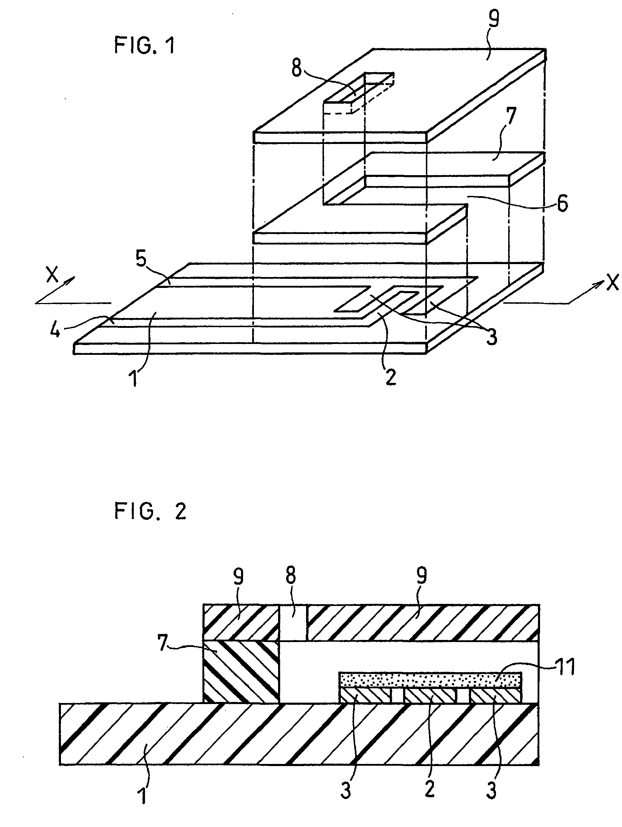

In the present example, in place of the electrochemical cell used in Example 1, a biosensor produced in the following procedure was used to conduct measurement in the same manner as in Example 1. In the present example, a biosensor with a structure shown in FIGS. 1 and 2 was produced.

FIG. 1 is an exploded perspective view of the biosensor used in the present example, from which a reagent system has been removed. A resin-made electrode pattern mask was placed on an glass-made electrically insulating base plate 1, and gold was sputtered to form a working electrode 2 and a counter electrode 3. It should be noted that a layer comprising chrome was formed as an adhesive layer between the gold and glass so that the adhesive property therebetween was enhanced. The working electrode 2 and counter electrode 3 were electrically connected to terminals for measurement outside the biosensor through means of leads 4 and 5, respectively.

After formation of a layer of a reagent system comprising oxidoreductase and an electron mediator on the working electrode 2, a spacer 7 having a slit 6 and a cover 9 having an air aperture 8 were bonded onto the base plate 1 in such a positional relationship as shown by the broken lines in FIG. 1, to produce a biosensor. A sample solution supply pathway was formed in the portion of the slit 6 in the spacer 7. The open end of the slit 6 at the end of the sensor was served as a sample supply opening for the sample solution supply pathway.

FIG. 2 is a vertical sectional view of a biosensor in accordance with the present invention. A reagent system 11 comprising oxidoreductase and an electron mediator was formed on the working electrode 2 formed on the base plate 1. As shown in FIG. 2, the reagent system 11 was formed on the electrode system comprising the working electrode 2 and counter electrode 3.

When a sample solution was brought into contact with the open end of the slit 6 to serve as the sample solution supply pathway of the sensor with the structure shown in FIG. 2, the sample solution was introduced into the sample solution supply pathway due to capillary action to dissolve the reagent system 11, and an enzyme reaction proceeded. Herein, as the sample solution supply pathway had previously been processed with an amphipathic reagent such as lecithin, the sample solution was introduced in a more uniform and smoother manner.

As thus described, when the base plate 1 where the electrode system was placed and the cover member, comprising the spacer 7 and the cover 9, were combined to form the sample solution supply pathway, between the base plate 1 and the cover member, for introducing the sample solution from the sample solution supply opening to the electrode system, the amount of the sample solution, containing the substrate as an object to be measured, to be supplied to the sensor could be fixed so that the precision of measurement could be improved.

In a sensor with the sample solution supply pathway provided therein, the reagent system might be disposed not only on the electrode system, but also in a portion exposed to the inside of the sample solution supply pathway in order that the reagent system would dissolve in a sample solution to be supplied. The reagent system might for example be provided in the portion of the cover 9 which was exposed to the inside of the sample solution supply pathway, and the portion not in contact with the electrode system on the base plate 1 but exposed to the inside of the sample solution supply pathway. Moreover, the reagent system might be divided into a plurality of portions, one of which might be provided on the base plate while the other be on the side of the cover member. In this regard, each of the divided layers was not necessarily required to contain all the reagents. For example, oxidoreductase and the electron mediator might be contained in separate layers.

Moreover, the insulating second base plate having uniting either the counter electrode 3 or the working electrode 4 with either the lead 5 or the lead 4 corresponding to the respective electrodes, might be used in place of the cover 9. Also in this case, since the base plate 1, the spacer 7 and the second base plate formed the sample solution supply pathway, the amount of the sample solution to be supplied to the sensor could be fixed so as to improve the precision of measurement.

The reagent system 11 was obtained by the use of ferrocene carboxylic acid and glucose oxidase, and 20 mM of a glucose aqueous solution as a sample solution, where a 0.5 mM ascorbic acid was dissolved, was dropped into the opening of the sample solution supply pathway of the sensor as thus produced, namely the open end of the slit 6 in the spacer 7, to be supplied to the sensor. After the lapse of a certain period of time, an AC potential, having a central potential of +0.1 V relative to the counter electrode 3 and the amplitude of 0.01 V, was applied to the working electrode 2. The AC potential had an equivalent frequency to that described in Example 1. After the lapse of another certain period of time, impedance Z was measured and complex impedance was plotted. As a result, as in Example 1, the plot appeared in the vicinity of the real number axis at a relatively low frequency; as the frequency was gradually increased, the magnitude of Z became smaller and the plot group formed an almost circular arc.

Next, as in the electrolyte described in Example 1, series of electrolytes with different glucose concentrations were prepared, and those electrolytes were separately supplied to the sensor to conduct measurement and plotting in the same manner as above. As the electrolytes with lower glucose concentrations were used, the diameter of the obtained circular arc gradually became longer, while having a certain correlation with the glucose concentration. Such a behavior was also observed in a case where a glucose aqueous solution containing no ascorbic acid was used as a sample solution.

It was therefore possible, by the same method as in Example 1, to determine the glucose aqueous solution concentration from the diameter of the circular arc of the obtained complex impedance plot, without the influence of ascorbic acid. It was also possible to estimate the glucose concentration by conducting the aforesaid alternating current measurement at a fixed single frequency and then using the magnitude of obtained Z, "|Z|". In this wise, the use of the method for quantitating a substrate in accordance with the present invention allowed precise quantitation of a substrate contained in a sample solution in a simply structured measurement system without occurrence of a measurement error due to an interfering substance.

Example 3

In the present example, an electrolyte (glucose concentration: 20 mM, ascorbic acid concentration: 0.5 mM) with the same composition as in Example 1 and an electrochemical cell were used. After the lapse of a certain period of time, an AC potential, which had a fixed single frequency, a central potential of + 0.1 V when taking the counter electrode as a reference and the amplitude of 0.01 V, was applied to the working electrode. After the lapse of a certain period of time, impedance Z was measured. Next, a DC potential of +0.3 V relative to the counter electrode was applied to the working electrode for a certain period of time to measure a direct current I' flowing between the working electrode and counter electrode.

As in Example 1, series of electrolytes with different glucose concentrations were used to conduct measurement and plotting in the same manner as above. As the electrolytes with lower glucose concentrations were used, obtained Z gradually became larger, having a certain correlation with the glucose concentration, as resulted in Example 1, and it was therefore possible to estimate the glucose concentration with the use of the Z value.

Separately from the above case, a DC potential of +0.3 V relative to the counter electrode was applied, or applied to the working electrode for a certain period of time, and then examined the relationship (I-G) between the direct current I and the glucose concentration under non-existence of ascorbic acid, as well as the relationship (I-A) between the direct current I and ascorbic acid under non-existence of glucose, by a method for measuring a direct current flowing between the working electrode and counter electrode. It was then revealed that each of I-G and I-A exhibited an almost favorable proportional relationship. On the other hand, it was found that each of the relationship between the direct current I and the glucose concentration under the existence of a fixed concentration of ascorbic acid, and the relationship between the direct current I and the ascorbic acid concentration under the existence of a fixed concentration of glucose exhibited an almost favorable liner relationship. It could thus be concluded that the direct current I' obtained by the aforesaid measurement was the sum of the respective currents brought about by glucose and ascorbic acid.

As thus described, it was possible to estimate the glucose concentration with the use of the Z value obtained by the alternating current measurement. When the glucose concentration as thus determined was applied to I-G, a current attributed to glucose among I' obtained by the aforesaid measurement could be found, and by deduction thereof, a current attributed to ascorbic acid among I' could be found. Application of this current to I-A could result in estimation of the ascorbic acid concentration.

As thus described, according to the method for quantitating a substrate in accordance with the present invention, quantitation of an interfering substance as well as measurement of a substrate could be conducted without the influence of the interfering substance. Respective analytical curves in respect to glucose and ascorbic acid based on a direct current, namely I-G and I-A, might not be prepared for every measurement, but be obtained previously.

It should be noted that in the present example, after the step of applying an AC potential to measure Z, the step of applying a DC potential to measure I' was implemented; however, the order of implementing these steps might be reversed. Further, the same measurement could be conducted by means of a biosensor, as in Example 2.

Example 4

In the present example, an electrochemical cell further comprising a reference electrode in an electrode system was used. The same electrolyte, working electrode and counter electrode were used as those in Example 1 and a silver/silver chloride electrode (Ag/AgCl electrode) was used as the reference electrode, to construct an electrochemical cell. A glucose aqueous solution as a sample solution where ascorbic acid was dissolved was applied in such a manner that the concentrations of ascorbic acid and glucose were 0.5 mM and 20 mM (about 400 mg/dL), respectively.

After the lapse of a certain period of time, an AC potential, which had a central potential of +0.36 V when taking the reference electrode as a reference and the amplitude of 0.01 V, was applied to the working electrode. A central potential at this working electrode was within the range of -0.1 V to +0.1 V relative to a redox potential of ferrocene carboxylic acid, and was a potential more positive than a potential that was 0.05 V negative (0.13 V (pH 7) vs Ag/AgCl) relative to the most negative potential (0.18 V (pH 7) vs Ag/AgCl) in a potential region where the reaction of ascorbic acid at the working electrode was diffusion-controlled. The same frequency of the AC potential as that in Example 1 was used.

After the lapse of another certain period of time, impedance Z was measured to plot complex impedance, and as in Example 1, the plot appeared on the real number axis at a relatively low frequency; as the frequency was gradually increased, the magnitude of Z became smaller and the plot group formed an almost circular arc.

Next, like the electrolytes described in Example 1, series of electrolytes with different glucose concentrations were prepared and these electrolytes were used to conduct measurement and plotting in the same manner as above. As the electrolytes with lower glucose concentrations were used, the diameter of the obtained circular arc gradually became longer, having a certain correlation with the glucose concentration. Such a behavior was also observed in a case where a glucose aqueous solution containing no ascorbic acid was used as a sample solution.

It was therefore possible, by the same method as in Example 1, to determine the glucose aqueous solution concentration from the diameter of the circular arc of the obtained complex impedance plot, without the influence of ascorbic acid. It was also possible to estimate the glucose concentration by conducting the aforesaid alternating current measurement at a fixed single frequency and then using the obtained magnitude of Z, |Z|.

Moreover, since the use of the reference electrode allowed more stable transfer of the potential of the working electrode, the obtained Z value was more stable compared with the case of Examples 1 and 2. In this wise, the method for quantitating a substrate in accordance with the present invention enabled the substrate measurement to be more stably implemented, without the influence of an interfering substance.

Furthermore, a sensor was produced in the same manner as in Example 2, and immediately after the supply of the sample solution containing ascorbic aid and glucose into the sensor, a silver/silver chloride electrode was brought into contact with the sample solution in the vicinity of the sample supply opening via a salt bridge comprising potassium chloride and agar. Except that an AC potential applied to the working electrode had a central potential of 0.36 V relative to the reference electrode and the amplitude of 0.01 V, an alternating current measurement was conducted in the same manner as in Example 2. Consequently, almost the same results as the results described in Example 2 were obtained. However, the obtained Z value was more stable. Hence, even in the case of using a reference electrode simultaneously with a sensor, according to the method for quantitating a substrate in accordance with the present invention, it was possible to conduct the substrate measurement in a stable manner without the influence of an interfering substance.

It should be noted that in the present example, although the silver/silver chloride electrode was brought into contact with the sample solution in the vicinity of the sample supply opening via the salt bridge, the similar effect can be obtained when the silver/silver chloride electrode was formed on the base plate of the sensor by screen-printing and then used.

Example 5

In the present example, first, 10 mL of a phosphate buffer with pH 7, where 1 mM of ruthenium hexacyanate and 0.6 kU/mL of pyloroquinoline quinone-dependent glucose dehydrogenase were dissolved as a reagent system, was obtained. This 10 mL phosphate buffer was put into a container made of Pyrex Glass to be used as an electrolyte, and a circular platinum disc with a diameter of 3 mm as a working electrode, a 2 cm-square platinum plate as a counter electrode, and an Ag/AgCl (sat.KCl) as a reference electrode were used to construct an electrochemical cell.

Subsequently, after the lapse of a certain period of time, an AC potential, having a central potential of +0.9 V relative to the reference electrode and the amplitude of 0.01 V, was applied to the working electrode. A central potential at this working electrode was within the range of -0.4 V to +0.4 V relative to a redox potential of ruthenium hexacyanate, and was a potential more positive than a potential that was 0.05 V negative (0.13 V (pH 7) vs Ag/AgCl) relative to the most negative potential (0.18 V (pH 7) vs Ag/AgCl) in a potential region where the reaction of ascorbic acid at the working electrode was diffusion-controlled. The frequency of the AC potential was successively varied from 16 mHz to 10 kHz, and more specifically, the same frequency as that shown in Example 1 was used.

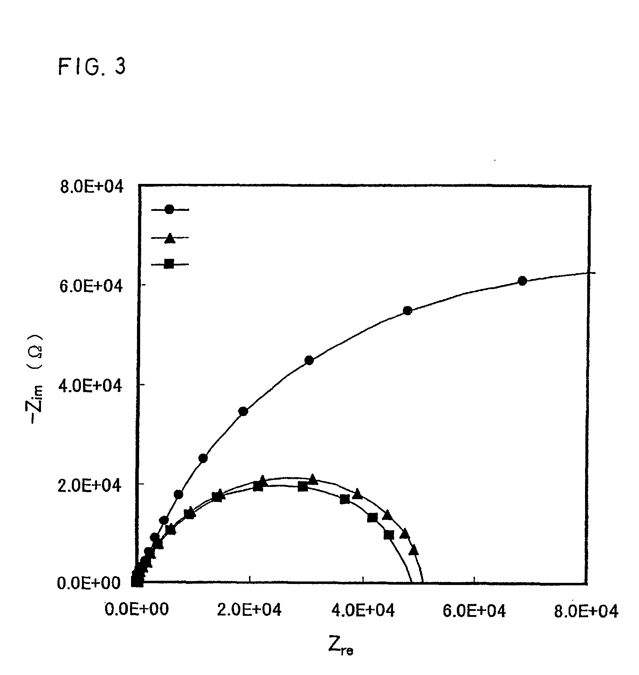

A certain period of time after the application of the AC potential, impedance Z was measured to plot complex impedance. As shown with ● in FIG. 3, in the absence of glucose, a plot forming almost a straight line was obtained. When a glucose aqueous solution as a sample solution was added such that the concentration thereof was 10 mM, as shown with ▲ in FIG. 3, the plot group appeared in the vicinity of the real number axis; as the frequency was gradually increased, the magnitude of Z became smaller and the plot group formed an almost circular arc.

Next, ascorbic acid was added into the aforesaid electrolyte such that the concentration thereof was 0.5 mM. Measurement and plotting were conducted in the same manner as above, to obtain the plot group showing almost the same half circle as in the case of adding 10 mM of the glucose aqueous solution, as shown with ■ in FIG. 3.

As thus described, even when a central potential of the AC potential to be applied to the working electrode was within the range of -0.4 V to +0.4 V relative to a redox potential of an electron mediator, and was a potential more positive than a potential that was 0.05 V negative relative to the most negative potential in a potential region where the reaction of the interfering substance at the working electrode was diffusion-controlled, the concentration of the glucose aqueous solution could be obtained from the diameter of the circular arc of the complex impedance plot group. Further, even when an AC potential having a central potential of 0.8 V, which was closer to the redox potential of ruthenium hexacyanate, was applied, the similar quantitation could be conducted. Moreover proved in the present example was that ruthenium hexacyanate functioned as a highly effective electron mediator in the present invention, and that pyloroquinoline quinone-dependent glucose dehydrogenase was a highly effective oxidoreductase in the present invention.

Example 6

In the present example, the same electrochemical cell as in Example 1 was used except that a platinum-made rotating disc electrode (RDE) was used as a working electrode, and the same sample solution and measurement conditions as in Example 1 were employed for measurement.

As a result, substantially, almost the same results as those in Example 1 were obtained; however, compared with the results of Example 1, the circular arc diameter was shorter, and a plot was obtained on the real number axis at a higher frequency. This was because the use of the RDE caused an increase in current variation derived from the electron mediator versus modulation of the potential applied to the working electrode, compared with the case of using a stationary electrode.

Next, like the electrolytes described in Example 1, series of electrolytes with different glucose concentrations were prepared, and used to conduct measurement and plotting in the same manner as above. As the electrolytes with the lower glucose concentrations were used, the diameter of the obtained circular arc gradually became longer, having a certain correlation with the glucose concentration. Such a behavior was also observed in a case where a glucose aqueous solution containing no ascorbic acid was used as a sample solution. Even with the RDE used instead of a stationary electrode, almost no influence on the amount of the current variation derived from the interfering substance versus the potential modulation was observed.

Accordingly, the glucose concentration could be measured with the diameter of this length. Worthy of note is that the variation in Z value versus the variation in glucose concentration was larger than the resulted valuation in Example 1. It was also possible to estimate the glucose concentration by conducting the alternating current measurement at a fixed single frequency and using the obtained Z value. Such an effect of using the RDE could also be obtained by using a stationary electrode and steadily stirring a solution which comprised a reagent system and a sample solution.

Further, almost the same result could be obtained in the case of conducting the same measurement as in the present example by using, in place of the RDE, a micro-electrode having an electrode area of such a size that a lateral diffusion of the electron mediator toward the electrode surface was contributed to the electrochemical reaction.

A micro-electrode of a preferable size was one having a radius within the range of 20 $\mu$m to about 50 $\mu$m and an electrode area within the range of 1000 to 8000 $\mu$m$^2$ when the shape thereof was converted into a circle. As the electrode employed could be one produced by putting carbon fiber, platinum, gold or the like into a glass capillary to be sealed and then making the surface of the electrode exposed, or a commercially available one. Further, a micro-electrode produced on a base plate by utilizing such a semiconductor processing step as photo lithography or etching could also be used. The use of such a micro-electrode was favorable especially when measurement was conducted using a biosensor as in Example 2.

As thus described, in the method for quantitating a substrate in accordance with the present invention, the use of an RDE or a micro-electrode as the working electrode made stable and highly sensitive substrate measurement possible.

It should be noted that in the above examples, the central potential of the AC potential to be applied to the working electrode was +0.1 V when taking the counter electrode as a reference, and +0.36 V or +0.9 V when taking the reference electrode as a reference; however, the central potential is not limited to these values and may be in the vicinity of a redox potential of an electron mediator, and a favorable value can be selected according to the type of electron mediator to be used. This value also varies depending on whether the reference electrode is used or not and a preferable value can be then selected.

Moreover, although the amplitude of the potential was 0.01 V in the above examples, it is not limited to this value, and any amplitude with which the alternating current measurement can substantially be conducted may be employed. While a value of usable amplitude is determined based on performance of a measurement instrument, a preferable value is 1 to 50 mV.

Although the frequency of the AC potential was 16 mHz to 10 kHz in the above examples, it is not limited to these values and range. Any frequency at which an electric signal obtained by the alternating current method significantly varies according to a substrate concentration may be employed.

Although the DC potential used in the above examples was +0.3 V when taking the counter electrode as a reference, it is not limited to this value; any potential with which an electron mediator and an interfering substance are sufficiently oxidized (or reduced) may be applied, and a favorable value can be selected according to the type of electron mediator to be used. Further, in the case of using the reference electrode, the DC potential can also vary depending on the type of reference electrode and a favorable value corresponding thereto is selected.

Furthermore, the electric signal obtained by the application of the DC potential was not limited to those described in the above examples. It may for example be a electrical charge passed at a working electrode.

The certain period of time for the reaction, which was used in the above examples, may be a period of time for which output of an observable degree can be obtained in almost a stable manner in implementation of the present invention.

Although platinum or gold was used as the electrode material in the above examples, it is not limited thereto. As other examples of electrodes using different materials cited can be an electrode comprising palladium and an electrode comprising carbon. Further, an electrode using a mixed material primarily comprising any one of platinum, gold, palladium and carbon can also be employed. Although the electrode comprising the working electrode and counter electrode, which comprised the equivalent material, was used in the above example, each electrode may be produced using different materials for the respective electrodes.

Although the spattering method through a mask was used as the production method of the electrodes in the biosensor as well as the production method of patterns of the electrodes in the above examples, the methods were not limited thereto, and for example, the pattern may be produced by combining a metal film formed by any of spattering, ion-plating, vapor deposition and chemical vapor deposition methods, with photo lithography and etching. The pattern can also be formed by trimming metal by means of a laser. The electrode pattern may also be formed by conducting screen-printing on a base plate with the use of a metal paste. Furthermore, patterned metal foil may be bonded onto a base plate as it is. When the electrode material is one mainly composed of carbon, the electrode pattern may be formed by conducting screen-printing on a base plate.

The shapes, dispositions, numbers, sizes and the like of these electrode systems are not limited to those described in the above examples. For example, a working electrode and a counter electrode may be formed on different insulating base plates, and a plurality of working electrodes and counter electrodes may be formed. The shape thereof may be comb-shaped. Further, the shapes, dispositions, number, sizes and the like of leads and terminals are not limited to those described in the above examples.

There is no limitation on the rotating rate of the RDE. Further, the electrode area of the micro-electrode may be within the range of such a size that a lateral diffusion of the electron mediator toward the electrode surface contributes to the electrochemical reaction.

The concentrations of the substrate and interfering substance in the sample solution are not particularly limited to those values described in the above examples. The sample solution amount is also not limited.

The amount or the concentration of each reagent contained in the reagent system is not limited to the values described in the above examples. It may be an amount with which an enzyme reaction and an electrochemical reaction proceed sufficiently.

An enzyme and an electron mediator may be in the insolubilized state or the non-eluted state by immobilizing, on the working electrode, the whole reagent system or one or more of reagents out of the reagents contained in the reagent system in the above examples. In the case of such immobilization, a covalent binding method, a cross-linking method, or a fixing method using interaction of coordinate bond and a specific affinity is preferably used. A method of surrounding an enzyme and an electron mediator by a polymer substance to give a pseudo-immobilized state is also effective as a method for readily forming a reagent system. A polymer to be used may be hydrophobic or hydrophilic, and the later is more preferable. As examples of hydrophilic polymers cited can be hydrophilic cellulose derivatives such as carboxy methyl cellulose, hydroxyethyl cellulose and ethyl cellulose, poly vinyl alcohol, gelatin, polyacrylic acid, starch and a derivative thereof, a maleic anhydride polymer, and a methacrylate derivative.

Further, it is more preferable that a pH buffer is contained in the reagent system in the above examples. This enables adjustment of pH of a reaction solution to be a suitable value for enzyme activity so that an enzyme can function effectively at the time of measurement. Moreover, since many interfering substances electrochemically react while involving a proton, a potential region where the reaction of the interfering substance is diffusion-controlled varies depending on pH of an electrolyte, but the use of the pH buffer allows the potential region to be fixed to certain values. It is therefore possible to stably remove the influence of the interfering substance in the present invention. As for the pH buffer used can be one containing one or more of phosphate, acetate, borate, citrate, phthalate and glycine. A buffer containing one or more of hydrogen salts out of the above salts may also be used. A reagent to be used as so-called "Good's buffer" may also be used. The forms of these pH buffers when contained within the sensor system can vary according to a sensor structure, and for example, they may be solid or solutions.

In the biosensors described in the above examples, it is preferable that a spacer is comprised as a constituent of the biosensor because the precision of measurement can be improved by readily and uniformly defining an amount of a solution containing a substrate as an object to be measured. However, in the case of using the biosensor described in the present invention in combination with an appliance capable of taking a fixed volume of a sample, a cover member comprising a spacer and a cover is not necessarily required.

Industrial Applicability

As thus described, according to the present invention, there can be provided a method capable of precisely quantitating a substrate contained in a sample solution by means of a measurement system with a simple structure, without occurrence of a measurement error due to an interfering substance.

**Claims**

1. A method for quantitating a substrate in a sample solution, which contains a dissolved interfering substance and said substrate, by the use of an electrode system and a reagent system, comprising the steps of:

   (a) supplying a sample solution which contains a dissolved interfering substance and a substrate to an electrode system comprising a working electrode and a counter electrode under the existence of a reagent system comprising oxidoreductase and an electron mediator;
   (b) applying an AC potential to said working electrode to cause a redox reaction of said electron mediator:
   (c) measuring an electric signal produced on the basis of said redox reaction, by means of said electrode system; and
   (d) quantitating said substrate on the basis of said electric signal.

2. The method for quantitating a substrate in accordance with Claim 1, **characterized in that** in said step (a), said working electrode and said counter electrode are disposed on the same plane.

3. The method for quantitating a substrate in accordance with Claim 1, **characterized in that** in said step (a), said working electrode and said counter electrode are disposed in positions opposed to each other across a space.

4. The method for quantitating a substrate in accordance with Claim 1, further comprising a step (e) of applying a DC potential to said working electrode, and a step (f) of measuring an electric signal produced in said step (e).

5. The method for quantitating a substrate in accordance with Claim 1, **characterized in that** in said step (b), a central potential of said AC potential is within the range of -0.4 to +0.4 V relative to a redox potential of said electron mediator, and is a potential more positive than a potential that is 0.05 V negative relative to the most negative potential in a potential region where the reaction of said interfering substance at said working electrode is diffusion-controlled.

6. The method for quantitating a substrate in accordance with Claim 1, **characterized in that** in said step (b), a central potential of said AC potential is within the range of -0.1 to +0.1 V relative to a redox potential of said electron mediator, and is a potential more positive than a potential that is +0.05 V relative to the most negative potential in a potential region where the reaction of said interfering substance at said working electrode is diffusion-controlled.

7. The method for quantitating a substrate in accordance with Claim 1, **characterized in that** said electric signal is impedance.

8. The method for quantitating a substrate in accordance with Claim 1, **characterized in that** said electrode system further comprises a reference electrode.

9. The method for quantitating a substrate in accordance with Claim 1, **characterized in that** said working electrode is a rotating disc electrode or a microelectrode.

10. The method for quantitating a substrate in accordance with Claim 1, **characterized in that** said oxidoreductase is glucose oxidase or pyrroloquinoline quinone-dependent glucose dehydrogenase, and said electron mediator is ferrocene carboxylic acid.

11. The method for quantitating a substrate in accordance with Claim 1, **characterized in that** said oxidoreductase is pyloroquinoline quinone-dependent glucose dehydrogenase, and said electron mediator is ruthenium hexacyanate.

FIG. 1

FIG. 2

FIG. 3

FIG. 4

FIG. 5

## INTERNATIONAL SEARCH REPORT

| | International application No. |
|---|---|
| | PCT/JP03/02613 |

| A. CLASSIFICATION OF SUBJECT MATTER |
|---|
| Int.Cl$^7$ G01N27/327 |
| |
| According to International Patent Classification (IPC) or to both national classification and IPC |

| B. FIELDS SEARCHED |
|---|
| Minimum documentation searched (classification system followed by classification symbols) |
| Int.Cl$^7$ G01N27/327 |

Documentation searched other than minimum documentation to the extent that such documents are included in the fields searched

| | | | |
|---|---|---|---|
| Jitsuyo Shinan Koho | 1922–1996 | Toroku Jitsuyo Shinan Koho | 1994–2003 |
| Kokai Jitsuyo Shinan Koho | 1971–2003 | Jitsuyo Shinan Toroku Koho | 1996–2003 |

Electronic data base consulted during the international search (name of data base and, where practicable, search terms used)
CAPLUS(STN)

### C. DOCUMENTS CONSIDERED TO BE RELEVANT

| Category* | Citation of document, with indication, where appropriate, of the relevant passages | Relevant to claim No. |
|---|---|---|
| X<br>Y | Susumu KUWABATA, Manobe SETANI, "Glucose Oxidase no Denkyoku Shokubai Hanno no Koryu Sokutei", The Electrochemical Society of Japan Taikai Koen Yoshishu, 25 March, 2001 (25.03.01), 68th, page 344 | 1,7,10<br>2,3,8,9,11 |
| Y | WO 97/39343 A1 (NUCLEARFUELS PLC.), 23 October, 1997 (23.10.97), Page 1<br>& GB 9607898 A  & AU 2644497 A<br>& CA 2251874 A  & EP 894265 A<br>& JP 2000-509488 A | 1,2,3,7,9-11 |
| Y | JP 9-159642 A (Dainippon Printing Co., Ltd.), 20 June, 1997 (20.06.97), Par. No. [0022]; Figs. 1, 6 (Family: none) | 1-3,7,8,10 |

| ☒ Further documents are listed in the continuation of Box C. | ☐ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 25 April, 2003 (25.04.03) | 13 May, 2003 (13.05.03) |

| Name and mailing address of the ISA/<br>Japanese Patent Office | Authorized officer |
|---|---|
| Facsimile No. | Telephone No. |

Form PCT/ISA/210 (second sheet) (July 1998)

**INTERNATIONAL SEARCH REPORT**

International application No.

PCT/JP03/02613

C (Continuation). DOCUMENTS CONSIDERED TO BE RELEVANT

| Category* | Citation of document, with indication, where appropriate, of the relevant passages | Relevant to claim No. |
|---|---|---|
| Y | "Bisho Denkyoku o Mochiiru Denki Kagaku Sokutei-ho", Satoshi IINO, 10 February, 1998 (10.02.98), pages 137, 138 | 9 |
| Y | ALVIN L.CRUMBLISS et al., THE ELECTROCHEMISTRY OF HEXACYANORUTHENATE AT CARBON ELECTRODES AND THE USE OF RUTHENIUM COMPOUNDS AS MEDIATORS IN THE GLUCOSE/GLUCOSE OXIDASE SYSTEM, Journal of Electroanalytical Chemistry and Interfacial Electrochemistry, 10 July, 1986 (10.07.86), Vol.206, No.1-2, pages 327 to 331 | 11 |
| A | JP 5-215709 A (NGK Spark Plug Co., Ltd.), 24 August, 1993 (24.08.93), Full text (Family: none) | 1-11 |
| A | WO 99/32881 A1 (ROCHE DIAGNOSTICS CORP.), 01 July, 1999 (01.07.99), Full text & AU 2089299 A & CA 2310021 A & EP 1042667 A & JP 2001-527215 A & NO 20003228 A | 1-11 |

Form PCT/ISA/210 (continuation of second sheet) (July 1998)